(19) Europäisches Patentamt
European Patent Office
Office européen des brevets

(11) **EP 3 301 434 A1**

(12) **EUROPEAN PATENT APPLICATION**

(43) Date of publication:
**04.04.2018 Bulletin 2018/14**

(51) Int Cl.:
**G01N 21/3504** *(2014.01)*  **G01N 33/00** *(2006.01)*
**G01N 33/22** *(2006.01)*  **G01N 21/27** *(2006.01)*
**G01N 21/03** *(2006.01)*  **G01N 21/31** *(2006.01)*

(21) Application number: **17193740.2**

(22) Date of filing: **28.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2016  US 201662402158 P**
**03.02.2017  US 201762454300 P**

(71) Applicant: **Ecotec Solutions, Inc.**
**Colton, CA 92324 (US)**

(72) Inventor: **JOURDAINNE, Laurent**
**Colton, California 92324 (US)**

(74) Representative: **De Clercq & Partners**
**Edgard Gevaertdreef 10a**
**9830 Sint-Martens-Latem (BE)**

(54) **COMPOSITION-INDEPENDENT CALIBRATION OF NONDISPERSIVE INFRARED GAS SENSORS**

(57) Systems and methods are disclosed for detecting the concentrations of constituent gases in a sample. Systems may include a sample chamber (206) configured to contain a volume of a gas, a light source (212) configured to emit infrared light through the volume of the gas, a reference channel, a plurality of active channels, and processing circuitry. The reference channel and the active channels can each include a bandpass optical filter (218, 222) configured to receive light from the light source (212) and selectively pass a portion of the light within a unique range of wavelengths, and a photodetector (220, 224) configured to detect infrared light from the light source (212) that passes through the bandpass optical filter (218, 222). The processing circuitry can be configured to calculate the concentrations within the sample gas of a plurality of constituent gases based at least in part on the light detected at each of the photodetectors.

FIG. 2A

## Description

INCORPORATION BY REFERENCE TO ANY PRIORITY APPLICATIONS

This application claims the benefit of U.S. Provisional Application Serial No. 62/402158, filed September 30, 2016, entitled "COMPOSITION-INDEPENDENT CALIBRATION OF NONDISPERSIVE INFRARED NATURAL GAS SENSORS," and U.S. Provisional Application Serial No. 62/454300, filed February 3, 2017, entitled "COMPOSITION-INDEPENDENT CALIBRATION OF NONDISPERSIVE INFRARED GAS SENSORS," both of which are hereby incorporated by reference in their entirety and for all purposes.

FIELD

This disclosure relates to gas composition analysis, and more specifically to systems and methods for improving the accuracy and calibration of nondispersive infrared gas sensors.

BACKGROUND

Natural gas leak detection systems and methods can include nondispersive infrared (NDIR) sensors. The effectiveness of existing NDIR sensors may be heavily dependent on the composition of the gas, for example, due to oversensitivity to methane and/or propane concentrations within the gas. Natural gases may contain various concentrations of methane, ethane, and/or propane depending on their origin. Thus, there exists a need for improved NDIR systems that can mitigate the oversensitivity to variations in gas composition and effectively detect gas mixtures independent of the relative concentrations of constituent gases within the gas.

SUMMARY

The systems and methods of this disclosure each have several innovative aspects, no single one of which is solely responsible for its desirable attributes. Without limiting the scope as expressed by the claims that follow, its more prominent features will now be discussed briefly.

In one embodiment, a nondispersive infrared gas detector is described. The gas detector includes a sample chamber configured to contain a volume of a sample gas, the sample gas including at least a first constituent gas and a second constituent gas, a light source configured to emit infrared light through the volume of the sample gas, a plurality of gas-specific channels, a reference channel, and processing circuitry. Each gas-specific channel includes a bandpass optical filter configured to receive light from the light source and selectively pass a portion of the light within a range of wavelengths centered on a wavelength $\lambda_n$ different from the

wavelength $\lambda_n$ of each of the other gas-specific channels, and a photodetector configured to detect infrared light from the light source that passes through the bandpass optical filter of the gas-specific channel. The reference channel includes a bandpass optical filter configured to receive light from the light source and selectively pass light within a range of wavelengths centered on a wavelength $\lambda_{ref}$ different from the wavelength $\lambda_n$ of each of the gas-specific channels, and a reference photodetector configured to detect infrared light from the light source that passes through the reference bandpass optical filter. The processing circuitry is configured to calculate the concentrations within the sample gas of the first constituent gas and the second constituent gas based at least in part on the light detected at the photodetectors of each of the gas-specific channels and the photodetector of the reference channel.

The first constituent gas and the second constituent gas can be hydrocarbon gases.

The first constituent gas can include methane, and the second constituent gas can include ethane.

The reference bandpass optical filter can be configured to selectively pass a range of wavelengths not associated with significant absorption by methane or ethane.

The sample gas can include a third constituent gas, and the processing circuitry can further be configured to calculate a third concentration of the third constituent gas within the sample gas based at least in part on the light detected at the photodetectors of each of the gas-specific channels and the photodetector of the reference channel.

A transmission coefficient of the reference bandpass optical filter can be within 5% of transmission coefficients of each of the bandpass optical filters of the gas-specific channels.

The nondispersive infrared gas detector can further include a plurality of waveguides disposed within the sample chamber, each waveguide extending along a longitudinal axis parallel to a path between the light source and at least one photodetector.

The light source can include a light emitting diode.

The light source can include a plurality of light emitting diodes, and a spectrum of infrared light emitted by each of the plurality of light emitting diodes can be different from a spectrum of infrared light emitted by at least another one of the plurality of light emitting diodes.

Each of the plurality of light emitting diodes can be associated with a gas-specific channel or the reference channel.

Each of the plurality of light emitting diodes can be operable independent of the other light emitting diodes.

The processing circuitry can be configured to calculate an absorbance of infrared light in each gas-specific channel based at least in part on a ratio of a light intensity measured at the photodetector of the gas-specific channel to a light intensity measured at the photodetector of the reference channel.

The processing circuitry can be configured to apply one or more absorbance models, each absorbance model corresponding to one of the gas-specific channels or the reference channel.

At least one of the absorbance models can be configured to calculate $f/(C_{gas}) = S(1 - \exp(\alpha \times C_{gas}{}^{\beta}))$, where $C_{gas}$ is a concentration of a constituent gas in the sample gas, and S, $\alpha$ and $\beta$ are predetermined constants determined during a calibration of the nondispersive infrared gas detector.

The nondispersive infrared gas detector can further include a gas inlet and a gas outlet in communication with the sample chamber, the gas inlet and the gas outlet configured to allow the sample gas to be cycled through the sample chamber.

BRIEF DESCRIPTION OF THE DRAWINGS

The above-mentioned aspects, as well as other features, aspects, and advantages of the present technology will now be described in connection with various implementations, with reference to the accompanying drawings. The illustrated implementations are merely examples and are not intended to be limiting. Throughout the drawings, similar symbols typically identify similar components, unless context dictates otherwise.

FIG. 1 is a schematic view of a nondispersive infrared (NDIR) gas sensor configuration having a gas-specific active channel and a reference channel in accordance with an exemplary embodiment.

FIG. 2A is a schematic view of an NDIR gas sensor configuration having a reference channel and a plurality of gas-specific active channels in accordance with an exemplary embodiment.

FIG. 2B is a schematic view of an alternative embodiment of an NDIR gas sensor configuration having a reference channel and a plurality of gas-specific active channels.

FIG. 3 depicts the infrared absorption spectra of example constituent gases and bandpass optical filter ranges in accordance with an exemplary embodiment.

DETAILED DESCRIPTION

The following description is directed to certain implementations for the purpose of describing the innovative aspects of this disclosure. However, a person having ordinary skill in the art will readily recognize that the teachings herein can be applied in a multitude of different ways. The described implementations may be implemented in any gas detection and/or analysis system.

Generally described, the systems and methods disclosed herein relate to improved systems and methods for detection and/or analysis of gases using nondispersive infrared (NDIR) detection systems. An active channel of an NDIR sensor, also referred to as a gas-specific channel, may include a bandpass optical filter configured to pass a range of wavelengths of infrared light including wavelengths that are heavily absorbed by a target gas, such as methane, for which a concentration is to be detected. The amount of light detected may be compared to the amount of light detected by a reference channel which is unfiltered or has an optical filter configured to pass wavelengths not absorbed by the target gas. However, other constituent gases in a mixed gas, such as natural gas, may also absorb a significant amount of light within the wavelength range passed by the bandpass optical filter of the active channel. Thus, an active channel configured to detect a particular gas may also be sensitive to other gases that are not intended to be measured. Furthermore, in some instances a detector may be overly sensitive to the presence of gases other than the target gas due to different infrared absorption characteristics of the gases. In the example context of natural gas, gases such as ethane and/or propane may absorb significantly more infrared light than methane, even in wavelength ranges in which methane has absorption peaks. Thus, while a single-channel NDIR detector may be accurate for detecting a concentration of methane in the absence of ethane and propane, a small amount of either ethane or propane in the mixed gas may produce a significant error in the detected concentration of methane. Furthermore, presence of ethane or propone in a single-channel NDIR detector configured to measure methane may not be detectable, thereby introducing errors in methane measurements that are unknown to the user.

As noted with reference to the example above, difficulties associated with oversensitivity to unwanted gases may be especially prevalent with gases containing constituent gases having similar absorption spectra. For example, natural gas may contain several simple hydrocarbon gases that may have partially overlapping absorption spectra (e.g., methane, ethane, propane, etc.). Accordingly, in some implementations, NDIR sensors may be implemented with additional active channels, each having a bandpass optical filter configured to pass a different range of wavelengths. An absorption may be detected for each active channel, and the relative concentrations of multiple constituent gases may be separately calculated based on the detected absorptions in the active channels. Additionally, the relationship between detection accuracy or sensitivity of a constituent gas may be adjusted (e.g., weighted) based on known impacts of a particular combination of constituent gases.

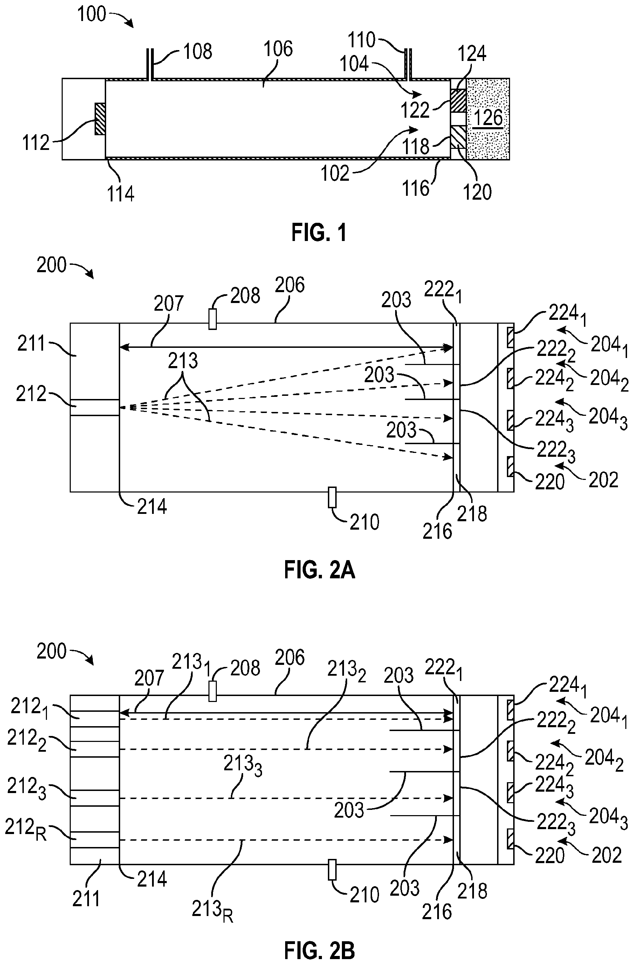

FIG. 1 is a schematic view of a simplified NDIR sensor 100 having a reference channel 102 and a single active channel 104. A sample chamber 106 includes a gas inlet 108 and a gas outlet 110 configured to allow a volume of gas to be cycled through the sample chamber. A light source 112, such as a light emitting diode (LED) or other light source, configured to emit infrared wavelengths is located at a first end 114 of the sample chamber 106 and positioned to emit infrared light across the volume of gas within the sample chamber 106. For example, the light source 112 may be configured to emit light in a range of wavelengths such as 750nm to 10,000nm, or any smaller range, such as 3,000nm to 4,000nm. The light source 112 may be selected to emit an appropriate range corresponding to absorption spectra of particular gases to be detected. For example, if the sensor 100 is to be used to detect natural gas that is known to likely include constituent gases such as methane, ethane, and propane, the light source 112 may be selected to emit light between 3,000nm and 4,000nm, where methane, ethane, and propane have significant infrared absorption peaks. In this example embodiment, the reference channel 102 and the active channel 104 are located at a second end 116 of the sample chamber 106 opposite the light source 112 at the first end 114.

The reference channel 102 can include an optical filter 118 and a photodetector 120. Similarly, the active channel 104 can include an optical filter 122 and a photodetector 124. For example, the optical filters 118, 122 may be bandpass filters configured to pass light having a desired range of wavelengths (e.g., the wavelength of the constituent gas to be measured), and/or to attenuate and/or block light having wavelengths outside the desired range. Each optical filter 118, 122 may be an absorptive filter, a dichroic filter, or other type of optical filter. The photodetectors 120, 124 of the reference channel and the active channel can be coupled to processing circuitry 126 or other hardware having software and/or firmware configured to receive data from the photodetectors 120, 124 and store, analyze, or otherwise process the received data.

In one example, the sensor 100 may be configured to detect a concentration of methane ($CH_4$) in the gas being cycled through the sample chamber 106 via the gas inlet 108 and the gas outlet 110. Accordingly, the bandpass optical filter 122 of the active channel 104 may be configured to pass light in a range of wavelengths containing absorption peaks in the infrared absorption spectrum of methane. For example, the optical filter 122 of the active channel 104 may be configured to selectively pass light having a wavelength between 3250nm and 3350nm, which includes most observed peaks of the infrared absorption spectrum for methane. The optical filter 118 of the reference channel 102 may be configured to pass light in a range of wavelengths in which methane does not heavily absorb infrared light, such as between 3350nm and 3450nm. The wavelengths selected in the reference channel 102 may be determined so as to pass light primarily in wavelengths not heavily absorbed by the expected constituent gases in the sample gas. Each optical filter 118, 122 of the reference channel 102 and the active channel 104 has a transmission coefficient, generally defined as the portion of the light incident on the filter that passes through and is not reflected or absorbed by the filter 188, 122, measured in terms of amplitude and/or intensity of the light. Preferably, the transmission coefficient of the reference channel filter 118 can be similar or equal to the transmission coefficient of the active channel filter 122 (e.g., within 5%, 10%, or similar), such that differences in detected absorption between the different photodetectors 120, 124 can be indicative of absorption by the sample gas without requiring additional correction for inconsistent absorption between the optical filters 118, 122. Thus, light may be emitted by the light source 112, and at least a portion of the light can be detected at the photodetectors 120, 124 of the active channel 102 and the reference channel 104. The intensities of light detected may be sent to the processing circuitry 126.

At the processing circuitry 126, an absorbance of the light in the active channel 104 may be determined using any suitable algorithm, such as mathematical operations consistent with Beer's Law or the like. For example, a number indicative of an intensity of light detected at the active channel 104 may be divided by a number indicative of an intensity of light detected at the reference channel 102 to determine an absorbance. The concentration of the constituent gas can then be determined based on the calculated absorbance of infrared light in the selected wavelength range passed by the active channel filter 122. In an example implementation, such a calculation can be performed using an equation of the form:

$$A = \log_{10}\left(\frac{\Phi^i}{\Phi^t}\right),$$

where A is the absorbance to be calculated, $\Phi^i$ is the radiant flux of the incident light emitted by the light source 112, and $\Phi^t$ is the radiant flux of the transmitted light which passes through to the photodetector 122. Because the wavelengths of light passed by the reference channel filter 118 are not absorbed by the sample gas, and transmission coefficients of the optical filters 118, 122 are substantially equal, the radiant flux of the emitted light in a gas-specific active channel 104 can be substantially equal to the radiant flux detected at the photodetector 120 of the reference channel 102. Thus, the absorbance equation given above can be evaluated for a gas-specific active channel 104 by taking the base-10 logarithm of the ratio of the radiant flux detected in the reference channel 102 to the radiant flux detected in the active channel 104. In a more concrete example, a reference channel 102 detects a radiant flux of 1 mW and a gas-specific active channel 104 detects a radiant flux of 0.99995 mW. The ratio $\Phi^i/\Phi^t$ would thus be approximately 1.00005. The absorbance A for the wavelength band passed by the optical filter 122 of the active channel 104 could then be calculated as $A = \log_{10}(1.00005)$, yielding an absorbance of $A = 2.17 \times 10^{-5}$. In various embodiments, smaller absorbances on the order of $10^{-6}$, $10^{-7}$, $10^{-8}$, $10^{-9}$, $10^{-10}$, or smaller may be accurately detected, based on the resolution of the photodetectors 120, 124.

A source of inaccuracy associated with detecting a concentration of a target gas in a mixed gas using the single active channel method described above may be the relative absorbance due to other gases present in the mixed gas. In one exemplary implementation, various natural gas compositions may contain a majority of methane, with ethane and propane comprising a smaller portion of the natural gas. For example, the methane concentration may range from 82% to 98% by volume, while ethane may range from 3% to 8% and propane may range from 0.5% to 2% by volume. However, in the wavelength ranges typically used for NDIR natural gas detection (e.g., infrared wavelengths), the absorbance associated with approximately 1% by volume of ethane may be equivalent to the absorbance associated with approximately 13% by volume of methane. The absorbance associated with approximately 5% by volume of ethane may be equivalent to the absorbance associated with approximately 100% by volume of methane. Thus, a relatively small ethane concentration in a natural gas sample can prevent such systems from accurately determining the methane concentration. This difficulty may be corrected for by calibration to take into account the greater absorbance by methane. However, such calibration is accurate only for a particular gas composition, making a calibrated single active channel sensor useful only for detecting a concentration of a particular gas composition.

FIG. 2A is a schematic view of an example multi-channel NDIR sensor 200 that may be used to detect concentrations of each of multiple constituent gases in a mixed gas. Similar to the sensor 100 depicted in FIG. 1, the sensor 200 includes a sample chamber 206 having a gas input 208 and a gas output 210, with an infrared light source 212 mounted in a light source mount 211 at a first end 214 of the sample chamber 206 and configured to emit light 213 in at least infrared wavelengths across the volume of gas within the sample chamber 206. The sensor 200 has an active channel 202 and a plurality of active channels $204_1$, $204_2$, and $204_3$ located at a second end 216 of the sample chamber 206 opposite the light source 212 at the first end 214. Waveguides 203 may extend along at least a portion of the sample chamber 206 to separate and/or guide the light from the light source 212 to the reference channel 202 and the active channels $204_1$, $204_2$, and $204_3$.

The reference channel 202 can include an optical filter 218 and a photodetector 220. Similarly, each active channel $204_1$, $204_2$, $204_3$ can include an optical filter $222_1$, $222_2$, $222_3$, respectively, and a photodetector $224_1$, $224_2$, $224_3$, respectively. For example, the optical filters 218, $222_1$, $222_2$, $222_3$ may be bandpass filters configured to pass light having a desired range of wavelengths, and/or to attenuate and/or block light having wavelengths outside the desired range. In the three-channel sensor 200 depicted, each of the three optical filters $222_1$, $222_2$, $222_3$ of the active channels $204_1$, $204_2$, $204_3$ may be configured to pass a different range of wavelengths, each range of wavelengths corresponding to a different one of three expected constituent gases. The optical filter 218 of the reference channel 202 may be configured to pass a range of wavelengths not heavily absorbed by the expected constituent gases associate with the active channels $204_1$, $204_2$, $204_3$.

The length 207 of the sample chamber 206 may be determined based on the known infrared absorbance of the target gas or gases and the expected concentrations of the target gases. For example, a target gas with a low infrared absorbance or a target gas expected to be found in low concentration within a mixed gas may require a longer sample chamber 206 than a target gas with a high infrared absorbance or a target gas expected to be found in a higher concentration, in order to provide an optical path between the light source 212 and the detector of sufficient length to produce a detectable amount of absorption at the detectors $224_1$, $224_2$, $224_3$ in the active channels $204_1$, $204_2$, $204_3$.

The example multi-channel sensor 200 depicted in FIG. 2A has a reference channel 202 and three active channels $204_1$, $204_2$, $204_3$, with each active channel $204_1$, $204_2$, $204_3$ and the reference channel 202 including a photodetector 220, $224_1$, $224_2$, $224_3$ and an optical filter 218, $222_1$, $222_2$, $222_3$ configured to attenuate at least a portion of the light 213 traveling from the infrared light source 212 to the photodetector 220, $224_1$, $224_2$, $224_3$ of the channel. For example, active channel $204_1$ may have an optical bandpass filter $222_1$ configured to selectively pass a range of wavelengths centered on a first wavelength $\lambda_1$, active channel $204_2$ may have an optical bandpass filter $222_2$ configured to selectively pass a range of wavelengths centered on a second wavelength $\lambda_2$, and active channel $204_3$ may have an optical bandpass filter $222_3$ configured to selectively pass a range of wavelengths centered on a third wavelength $\lambda_3$. Although the sensor 200 depicted in FIG. 2A includes a reference channel 202 and three active channels $204_1$, $204_2$, $204_3$, any number of active channels may equally be used in accordance with the embodiments described herein. For example, the sensor may include 2, 3, 4, 5, or more active channels.

Similar to the single active channel sensor 100 depicted in FIG. 1, the photodetectors 220, $224_1$, $224_2$, $224_3$ of the active and reference channels 202, $204_1$, $204_2$, $204_3$ may be coupled to processing circuitry or other hardware having software and/or firmware configured to receive data from the photodetectors and store, analyze, or otherwise process the received data. As will be described in greater detail below, the processing circuitry may calculate an absorbance for each of the active channels $204_1$, $204_2$, $204_3$ relative to the reference channel 202 as described above. The absorbances calculated for each wavelength range may then be used to determine a relative concentration of various constituent gases. For example, in some embodiments, the determination of relative concentrations may be achieved by solving a system of equations, such as by matrix calculations, and the number of constituent gases that can be analyzed may be equal to the number of active channels $204_1$, $204_2$, $204_3$ of the sensor 200.

FIG. 2B depicts an alternative configuration of an example multi-channel NDIR sensor 200 that may be used to detect the concentration of various gases within a mixed gas. Similar to the configuration depicted in FIG. 2A, the sensor 200 of FIG. 2B includes a sample chamber 206 having a gas input 208 and a gas output 210, a reference channel 202, and a plurality of active channels $204_1$, $204_2$, $204_3$, the reference channel 202 and the active channels $204_1$, $204_2$, $204_3$ each including a detector 220, $224_1$, $224_2$, $224_3$. However, the embodiment depicted in FIG. 2B includes multiple infrared light sources $212_1$, $212_2$, $212_3$, $212_R$. For example, the sensor can include one infrared light source for each channel. In the example shown in FIG. 2B, light source $212_R$ produces a reference beam $213_R$ for the reference channel 202, light source $212_1$ produces a beam $213_1$ for the active channel $204_1$, light source $212_2$ produces a beam $213_2$ for the active channel $204_2$, and light source $212_3$ produces a beam $213_3$ for the active channel $204_3$.

Including a plurality of infrared light sources $212_1$, $212_2$, $212_3$, $212_R$ within the sample chamber 206 can provide several advantages. In one aspect, several light sources $212_1$, $212_2$, $212_3$, $212_R$ may provide a more uniform distribution of infrared light, reducing any error in the detected absorption at the various channels due to special disposition relative to a single light source. In another aspect, the use of multiple light sources $212_1$, $212_2$, $212_3$, $212_R$ may allow for a customized spectrum in each channel or a more finely tuned spectrum across all channels. For example, a particular mixed gas composition may be best analyzed using a spectrum of infrared light that cannot be generated by a single available light source. However, a desired spectrum may be achievable by a combination of light sources, each configured to produce a different spectrum of infrared light.

In another example, the plurality of infrared light sources $212_1$, $212_2$, $212_3$, $212_R$ may be independently operable, such that a sample gas may first be analyzed under a first spectrum of infrared light (e.g., only a first one of the multiple infrared light sources $212_1$, $212_2$, $212_3$, $212_R$ is powered), and may subsequently be analyzed under one or more different spectra of infrared light (e.g., only a second one of the multiple infrared light sources $212_1$, $212_2$, $212_3$, $212_R$ is powered). In yet another example, each waveguide 203 may extend the full length 207 of the sample chamber 206 such that each channel 202, $204_1$, $204_2$, $204_3$ is illuminated by a separate light source $212_1$, $212_2$, $212_3$, $212_R$ without mixing of light from multiple infrared emitters. In such embodiments, each channel 202, $204_1$, $204_2$, $204_3$ may have an identical filter 218, $222_1$, $222_2$, $222_3$ such that the spectrum detected at each detector is determined based on the tuning of the light sources $212_1$, $212_2$, $212_3$, $212_R$, rather than the bands passed by the filter 218, $222_1$, $222_2$, $222_3$. In other embodiments, each channel 202, $204_1$, $204_2$, $204_3$ may have a different optical filter 218, $222_1$, $222_2$, $222_3$ and an identically or differently tuned infrared light source $212_1$, $212_2$, $212_3$, $212_R$.

In any of these embodiments, selective activation of various light sources $212_1$, $212_2$, $212_3$, $212_R$ may be automatically controlled by a computing system (such as a programmed microcontroller). For example, a sensor system (e.g., a sensor and an associated microcontroller) may be configured to automatically cycle the infrared sensors repeatedly to obtain the corresponding measurements from one or more photo detectors. In one example, the sensor system may include four infrared light sources $212_1$, $212_2$, $212_3$, $212_R$ that are alternatively activated for x seconds each (e.g., <1 second to multiple seconds) to obtain various readings.

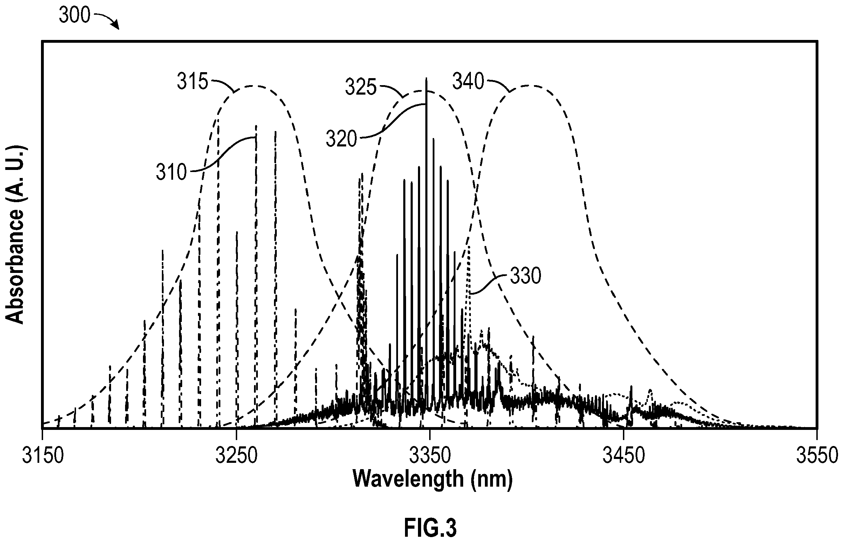

FIG. 3 depicts an example spectrum 300 illustrating a combination of optical bandpass filter wavelength ranges that may be used in accordance with the systems and methods described herein. In the example spectrum 300, each of methane, ethane, and propane has absorption peaks at various wavelengths within the spectrum between 3150nm and 3550nm. The wavelengths $\lambda_n$ of the active and reference channels may be determined based on the absorption spectra of the constituent gases expected to be present. For example, the depicted combination of spectra includes an absorption spectrum 310 corresponding to methane, an absorption spectrum 320 corresponding to ethane, and an absorption spectrum 330 corresponding to propane. Thus, in a multi-channel sensor such as the sensor 200 described with reference to FIGS. 2A and 2B, a first active channel light source $212_1$ and/or optical filter $222_1$ may have a wavelength range 315 centered at approximately 3250nm, where absorption is primarily by methane as shown by the peaks of the methane absorption spectrum 310. A second active channel light source $212_2$ and/or optical filter $222_2$ may have a wavelength range 325 centered at approximately 3350nm, where absorption is primarily by ethane as shown by the peaks of the ethane absorption spectrum 320. A reference channel light source $212_R$ and/or optical filter 218 may have a wavelength range 340 centered at a higher wavelength, such as approximately 3400nm, where neither methane nor ethane absorbs a significant amount of light, or may be centered higher (e.g., 3500nm) where propane that may be present would not interfere with the reference reading.

For each frequency range 315, 325, 340, an absorbance function $A_n$ may be calculated based on the known absorption characteristics of the constituent gases to be measured, methane and ethane in this example, and/or other possibly present gases such as propane. The set of absorbance functions may be combined to form a solvable system of equations. For example, in an exemplary embodiment configured to detect constituent gases of natural gas, such a system may be of the form:

$$A_1 = f(C_{methane}, C_{ethane}, C_{propane})$$

$$A_2 = g(C_{methane}, C_{ethane}, C_{propane})$$

$$A_3 = h(C_{methane}, C_{ethane}, C_{propane}),$$

where A is an absorbance function, $C_{methane}$, $C_{ethane}$, and $C_{propane}$ are variables representing the respective concentrations of methane, ethane, and propane, and $f$, $g$, and $h$ are predetermined functions based on the known relative absorbances due to each constituent gas in the known wavelength ranges of each optical filter. In some aspects, the absorbance functions may have a general form such as:

$$f(C_{methane}) = S(1 - \exp(\alpha \times C_{methane}^{\beta})),$$

where $C_{methane}$ is the concentration of methane in the sample gas, and S, $\alpha$ and $\beta$ are additional constants dependent on the gas and sensor design. In some aspects, the constants S, $\alpha$ and $\beta$ may be determined experimentally for an individual detector 200 and/or for a group of similarly designed detectors 200 before use, such as during a calibration or testing process. Any number of absorbance functions may be calculated, depending on the number of active channels $204_1$, $204_2$, $204_3$, etc., used in the sensor 200. In addition, one of the absorbance functions may be an absorbance function for the reference channel 202. Once the absorbance functions are known, data may be collected by sending infrared light through a gas sample, setting each absorbance function equal to the detected absorbance at the associated photodetector 220, $224_1$, $224_2$, $224_3$, and solving the system of equations to determine the values of $C_{methane}$, $C_{ethane}$, and $C_{propane}$, and/or the concentrations of any other expected constituent gases within the sample.

Determining the relative concentrations of the constituent gases in a mixed gas can facilitate several advantageous implementations. In one example, the calibration systems and methods described herein can be used to improve the accuracy of the detection of a single target gas. For example, where the concentration of methane is to be detected in a mixture of natural gas and air (e.g., where possible constituents of natural gas that may be present in the air is not known), the absorption contributions due to ethane and propane in the natural gas can accurately be corrected for, and a more precise concentration of methane can be determined.

In another example, the systems and methods described herein can be used to determine the relative concentrations of an unknown mixture of gases. For example, in the case of natural gas leak detection, it may be known that a mixture of air and natural gas will include methane, ethane, and propane. A sensor having active channels tuned to detect methane, ethane, and propane as described herein may be configured both to detect the presence of such gases, as well as to provide an indication of the relative concentrations of the gases within detected natural gas. In some instances, such a categorization of the constituent gases may be useful for identifying the source of a gas leak, such as where a plurality of natural gas compositions are present in the same vicinity.

In general, the microprocessors and/or computing discussed herein may each include on or more "components" or "modules," wherein generally refer to logic embodied in hardware or firmware, or to a collection of software instructions, possibly having entry and exit points, written in a programming language, such as, for example, Java, Lua, C or C++. A software module can be compiled and linked into an executable program, installed in a dynamic link library, or can be written in an interpreted programming language such as, for example, BASIC, Perl, or Python. It will be appreciated that software modules can be callable from other modules or from themselves, and/or can be invoked in response to detected events or interrupts. Software modules configured for execution on computing devices can be provided on a computer readable medium, such as a compact disc, digital video disc, flash drive, magnetic disc, or any other tangible medium, or as a digital download (and can be originally stored in a compressed or installable format that requires installation, decompression or decryption prior to execution). Such software code can be stored, partially or fully, on a memory device of the executing computing device, for execution by the computing device. Software instructions can be embedded in firmware, such as an EPROM. It will be further appreciated that hardware modules can be comprised of connected logic units, such as gates and flip-flops, and/or can be comprised of programmable units, such as programmable gate arrays or processors. The modules or computing device functionality described herein are preferably implemented as software modules, but can be represented in hardware or firmware. Generally, the modules described herein refer to logical modules that can be combined with other modules or divided into sub-modules despite their physical organization or storage.

The term "non-transitory media," and similar terms, as used herein refers to any media that store data and/or instructions that cause a machine to operate in a specific fashion. Such non-transitory media can comprise non-volatile media and/or volatile media. Non-volatile media includes, for example, optical or magnetic disks, such as storage device. Volatile media includes dynamic memory, such as main memory. Common forms of non-transitory media include, for example, a floppy disk, a flexible disk, hard disk, solid state drive, magnetic tape, or any other magnetic data storage medium, a CD-ROM, any other optical data storage medium, any physical medium with patterns of holes, a RAM, a PROM, and EPROM, a FLASH-EPROM, NVRAM, any other memory chip or cartridge, and networked versions of the same.

The foregoing description details certain embodiments of the systems, devices, and methods disclosed herein. It will be appreciated, however, that no matter how detailed the foregoing appears in text, the devices and methods can be practiced in many ways. As is also stated above, it should be noted that the use of particular terminology when describing certain features or aspects of the invention should not be taken to imply that the terminology is being re-defined herein to be restricted to including any specific characteristics of the features or aspects of the technology with which that terminology is associated. The scope of the disclosure should therefore be construed in accordance with the appended claims and any equivalents thereof.

With respect to the use of any plural and/or singular terms herein, those having skill in the art can translate from the plural to the singular and/or from the singular to the plural as is appropriate to the context and/or application. The various singular/plural permutations may be expressly set forth herein for sake of clarity.

It is noted that the examples may be described as a process. Although the operations may be described as a sequential process, many of the operations can be performed in parallel, or concurrently, and the process can be repeated. In addition, the order of the operations may be rearranged. A process is terminated when its operations are completed. A process may correspond to a method, a function, a procedure, a subroutine, a subprogram, etc.

The previous description of the disclosed implementations is provided to enable any person skilled in the art to make or use the present disclosed process and system. Various modifications to these implementations will be readily apparent to those skilled in the art, and the generic principles defined herein may be applied to other implementations without departing from the spirit or scope of the disclosed process and system. Thus, the present disclosed process and system is not intended to be limited to the implementations shown herein but is to be accorded the widest scope consistent with the principles and novel features disclosed herein.

**Claims**

1. A nondispersive infrared gas detector comprising:

   a sample chamber configured to contain a volume of a sample gas, the sample gas comprising at least a first constituent gas and a second constituent gas;
   a light source configured to emit infrared light through the volume of the sample gas;
   a plurality of gas-specific channels, each gas-specific channel comprising:

   a bandpass optical filter configured to receive light from the light source and selectively block a portion of the light outside a range of wavelengths centered on a wavelength $\lambda_n$ different from the wavelength $\lambda_n$ of each of the other gas-specific channels; and
   a photodetector configured to detect infrared light from the light source that passes through the bandpass optical filter of the gas-specific channel;

   a reference channel comprising:

   a reference bandpass optical filter configured to receive light from the light source and selectively block a portion of the light outside a range of wavelengths centered on a wavelength $\lambda_{ref}$ different from the wavelengths $\lambda_n$ of all of the gas-specific channels; and
   a reference photodetector configured to detect infrared light from the light source that passes through the reference bandpass optical filter; and

   processing circuitry configured to calculate:

   a first concentration of the first constituent gas within the sample gas based at least in part on the light detected at the photodetectors of each of the gas-specific channels and the photodetector of the reference channel; and
   a second concentration of the second constituent gas within the sample gas based at least in part on the light detected at the photodetectors of each of the gas-specific channels and the photodetector of the reference channel.

2. The nondispersive infrared gas detector of Claim 1, wherein the first constituent gas and the second constituent gas are hydrocarbon gases.

3. The nondispersive infrared gas detector of Claim 1, wherein the first constituent gas comprises methane, and wherein the second constituent gas comprises ethane.

4. The nondispersive infrared gas detector of Claim 3, wherein the reference bandpass optical filter is configured to selectively pass a range of wavelengths not associated with significant absorption by methane or ethane.

5. The nondispersive infrared gas detector of Claim 3, wherein the sample gas comprises a third constituent gas, and wherein the processing circuitry is further configured to calculate a third concentration of the third constituent gas within the sample gas based at least in part on the light detected at the photodetectors of each of the gas-specific channels and the photodetector of the reference channel.

6. The nondispersive infrared gas detector of Claim 1, wherein a transmission coefficient of the reference bandpass optical filter is within 5% of transmission coefficients of each of the bandpass optical filters of the gas-specific channels.

7. The nondispersive infrared gas detector of Claim 1, further comprising a plurality of waveguides disposed within the sample chamber, each waveguide extending along a longitudinal axis parallel to a path between the light source and at least one photodetector.

8. The nondispersive infrared gas detector of Claim 1, wherein the light source comprises a light emitting diode.

9. The nondispersive infrared gas detector of Claim 1, wherein the light source comprises a plurality of light emitting diodes, and wherein a spectrum of infrared light emitted by each of the plurality of light emitting diodes is different from a spectrum of infrared light emitted by at least another one of the plurality of light emitting diodes.

10. The nondispersive infrared gas detector of Claim 9, wherein each of the plurality of light emitting diodes is associated with a gas-specific channel or the reference channel.

11. The nondispersive infrared gas detector of Claim 9, wherein each of the plurality of light emitting diodes is operable independent of the other light emitting diodes.

12. The nondispersive infrared gas detector of Claim 1, wherein the processing circuitry is configured to calculate an absorbance of infrared light in each gas-specific channel based at least in part on a ratio of a light intensity measured at the photodetector of the gas-specific channel to a light intensity measured at the photodetector of the reference channel.

13. The nondispersive infrared gas detector of Claim 1, wherein the processing circuitry is configured to apply one or more absorbance models, each absorbance model corresponding to one of the gas-specific channels or the reference channel.

14. The nondispersive infrared gas detector of Claim 13, wherein at least one of the absorbance models is configured to calculate $f(C_{gas}) = S(1 - \exp(\alpha \times C_{gas}^{\beta}))$, where $C_{gas}$ is a concentration of a constituent gas in the sample gas, and S, $\alpha$ and $\beta$ are predetermined constants determined during a calibration of the nondispersive infrared gas detector.

15. The nondispersive infrared gas detector of Claim 1, further comprising a gas inlet and a gas outlet in communication with the sample chamber, the gas inlet and the gas outlet configured to allow the sample gas to be cycled through the sample chamber.

**FIG. 1**

**FIG. 2A**

**FIG. 2B**

FIG.3

Europäisches
Patentamt

European
Patent Office

Office européen
des brevets

# EUROPEAN SEARCH REPORT

Application Number

EP 17 19 3740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUN YOU-WEN ET AL: "Cross-interference correction and simultaneous multi-gas analysis based on infrared absorption", CHINESE PHYSICS B, CHINESE PHYSICS B, BRISTOL GB, vol. 21, no. 9, 13 September 2012 (2012-09-13), page 90701, XP020229006, ISSN: 1674-1056, DOI: 10.1088/1674-1056/21/9/090701 | 1-5,12, 15 | INV. G01N21/3504 G01N33/00 G01N33/22 G01N21/27 G01N21/03 G01N21/31 |
| Y | * page 3, right-hand column, lines 1-14 * * page 3, left-hand column, lines 11-14, paragraphs 2,3 * * page 4, left-hand column, paragraph 1 * * equations 3,4 * * figure 1 * | 1-15 | |
| | ----- | | |
| Y | US 7 132 657 B2 (SENSOR ELECTRONICS CORP [US]) 7 November 2006 (2006-11-07) * column 1, lines 6,67 * * column 2, lines 33-37,38-49 * * column 4, lines 42,43 * * column 5, lines 42,43,56-58 * * column 6, lines 4-6, 12-14 * * column 8, lines 1,2,13-16,61 * * column 9, lines 64,65 * * column 10, lines 4,5,61,64 * * figures 2,3,4,6 * | 1-5,7,8, 12,13,15 | |
| | | | TECHNICAL FIELDS SEARCHED (IPC) |
| | | | G01N |
| | ----- | | |
| Y | US 4 507 558 A (BONNE ULRICH [US]) 26 March 1985 (1985-03-26) * column 1, lines 49-51,56-57,59-60 * * column 2, lines 4,5-7,50-52,60-62 * * column 3, lines 8-16, 43 - 47 * * column 4, lines 30-32,41,42 * * figure 1 * | 1-4, 8-12,15 | |
| | ----- | | |
| | -/-- | | |

The present search report has been drawn up for all claims

| Place of search | Date of completion of the search | Examiner |
|---|---|---|
| Munich | 12 February 2018 | Brauer, Jan |

EPO FORM 1503 03.82 (P04C01)

page 1 of 2

Europäisches
Patentamt

European
Patent Office

Office européen
des brevets

**EUROPEAN SEARCH REPORT**

Application Number

EP 17 19 3740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 277 081 B1 (SUSI ROGER [US] ET AL) 21 August 2001 (2001-08-21) <br> * column 1, lines 5-8 * <br> * column 4, lines 29-32,39-42 * <br> * column 9, lines 37-39 * <br> * equations 13 * <br> * figure 5 * <br> ----- | 1,12-15 | |
| Y | Kosmas Galatsis ET AL: "Car Cabin Air Quality Sensors and Systems", EOS, 31 December 2006 (2006-12-31), XP055442725, Retrieved from the Internet: URL:http://www.co-gas-expert.com/wp-content/uploads/2012/12/Encyclopedia_Chapter.pdf [retrieved on 2018-01-19] <br> * page 7, paragraph 2 * <br> * figure 15 * <br> ----- | 6 | |
| Y | US 2011/042570 A1 (WONG JACOB Y [US]) 24 February 2011 (2011-02-24) <br> * paragraph [0039] * <br> * figure 3 * <br> ----- | 7 | **TECHNICAL FIELDS SEARCHED** (IPC) |

The present search report has been drawn up for all claims

| Place of search | Date of completion of the search | Examiner |
|---|---|---|
| Munich | 12 February 2018 | Brauer, Jan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT**
**ON EUROPEAN PATENT APPLICATION NO.**                    EP 17 19 3740

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2018

| Patent document cited in search report | | Publication date | Patent family member(s) | | Publication date |
|---|---|---|---|---|---|
| US 7132657 | B2 | 07-11-2006 | NONE | | |
| US 4507558 | A | 26-03-1985 | NONE | | |
| US 6277081 | B1 | 21-08-2001 | AU | 4855100 A | 05-12-2000 |
| | | | EP | 1181532 A1 | 27-02-2002 |
| | | | US | 6277081 B1 | 21-08-2001 |
| | | | WO | 0070330 A1 | 23-11-2000 |
| US 2011042570 | A1 | 24-02-2011 | AU | 2010284205 A1 | 15-03-2012 |
| | | | CA | 2771627 A1 | 24-02-2011 |
| | | | EP | 2478345 A1 | 25-07-2012 |
| | | | US | 2011042570 A1 | 24-02-2011 |
| | | | WO | 2011022558 A1 | 24-02-2011 |

EPO FORM P0459

For more details about this annex : see Official Journal of the European Patent Office, No. 12/82

**EP 3 301 434 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62402158 A
- US 62454300 A